(19) Europäisches Patentamt
European Patent Office
Office européen des brevets

(11) Veröffentlichungsnummer: **0 012 271**
**B1**

(12) # EUROPÄISCHE PATENTSCHRIFT

(45) Veröffentlichungstag der Patentschrift: **02.05.84**

(51) Int. Cl.³: **C 07 J 7/00, A 61 K 31/57**

(21) Anmeldenummer: **79104733.5**

(22) Anmeldetag: **28.11.79**

(54) **Neue Prednison-Derivate, Verfahren zu ihrer Herstellung, diese Verbindungen enthaltende pharmazeutische Präparate und deren Verwendung.**

(30) Priorität: **11.12.78 DE 2853785**

(43) Veröffentlichungstag der Anmeldung:
**25.06.80 Patentblatt 80/13**

(45) Bekanntmachung des Hinweises auf die Patenterteilung:
**02.05.84 Patentblatt 84/18**

(84) Benannte Vertragsstaaten:
**AT BE CH DE FR GB IT LU NL SE**

(56) Entgegenhaltungen:
**FR - A - 2 366 313**
**FR - A - 2 384 792**
**US - A - 2 973 356**
**US - A - 3 832 366**

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Annen, Klaus, Dr.**
**Seegefelder Strasse 194**
**D-1000 Berlin 20 (DE)**
Erfinder: **Laurent, Henry, Dr.**
**Glambecker Weg 21**
**D-1000 Berlin 28 (DE)**
Erfinder: **Hofmeister, Helmut, Dr.**
**Weislingenstrasse 4**
**D-1000 Berlin 28 (DE)**
Erfinder: **Kapp, Joachim-Friedrich, Dr.**
**Ludolfinger Weg 51**
**D-1000 Berlin 28 (DE)**
Erfinder: **Wendt, Hans, Dr.**
**Ernst-Ring-Strasse 3**
**D-1000 Berlin 38 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Prednison-Derivate, Verfahren zu ihrer Herstellung, diese Verbindungen enthaltende pharmazeutische Präparate und deren Verwendung

Die Erfindung betrifft neue Prednison-Derivate der allgemeinen Formel I gemäß Anspruch 1, ein Verfahren zu ihrer Herstellung, pharmazeutische Präparate, die Prednison-Derivate als Wirkstoff enthalten und deren Verwendung.

Unter einer bis zu 8 Kohlenstoffatome enthaltenden Alkylgruppe der allgemeinen Formel I soll eine geradkettige oder verzweigte Alkylgruppe (wie zum Beispiel die Methylgruppe, die Äthylgruppe, die Propylgruppe, die Isopropylgruppe, die Butylgruppe, die Isobutylgruppe, die tert.-Butylgruppe, die Pentylgruppe, die Hexylgruppe, die Heptylgruppe oder die Octylgruppe) verstanden werden.

Das erfindungsgemäße Verfahren zur Herstellung der neuen Prednison-Derivate kann beispielsweise unter den Bedingungen durchgeführt werden, die in den US-Patentschriften 3.678.034, 3.718.671, 3.828.083, 3.992.422 und 4.021.459 beschrieben sind.

Die Prednison-Derivate der allgemeinen Formel I gemäß Anspruch 1 zeichnen sich gegenüber dem aus der französischen Patentschrift 23 66 313 vorbekannten 9$\alpha$,21-Dichlor-11$\beta$-hydroxy-17$\alpha$-propionyloxy-1,4-pregnadien-3,20-dion, dem aus der US-Patentschrif 29 73 356 vorbekannten 9$\alpha$,21-Difluor-17$\alpha$-hydroxy-1,4-pregnadien-3,11,20-trion und dem aus der US-Patentschrift 38 32 366 vorbekannten und in handelsüblichen pharmazeutischen Präparate befindlichen, strukturanalogen Kortikoid 17$\alpha$-Butyryloxy-21-chlor-9$\alpha$-fluor-16$\beta$-methyl-1,4-pregnadien-3,11,20-trion durch eine günstigere Dissoziation zwischen erwünschter topischer antiinflammatorischer Wirksamkeit und unerwünschter systemischer Nebenwirkung aus, wie folgende pharmakologische Untersuchungen zeigen:

a) Ermittlung der topischen antiinflammatorischen Wirksamkeit:

Die Untersuchungen werden an je 8 gesunden Probanden beider Geschlechter durchgeführt, die in den letzten zwei Wochen keine lokale Corticosteroidbehandlung erhalten hatten. Nach dem Entfernen des Stratum corneum bis zum Stratum lucidum auf dem Rücken der Probanden (20—40 Tesafilm-Abrisse) werden je o,l g der Zubereitung auf 4 $cm^2$ große Felder ohne Okklusionsverband aufgetragen. Um zu vermeiden, daß die gleiche Zubereitung jeweils auf identische Hautreale appliziert wird, wird in rotierender Folge aufgetragen.

Die Vasokonstriktion wird visuell nach 4 und 8 Stunden durch den Prüfer nach folgenden Wirkungsgraden beurteilt: 1 = absolute Abblassung, 2 = geringes Resterythem, 3 = mittelgradiges Erythem, Rötungsintensität im mittleren Bereich von gestrippter, unbehandelter und nicht geschädigter Haut, 4 = Erythem mit geringen Aufhellungen, 5 = keine Abblassung oder Verstärkung des Erythems.

Die Einzelbeurteilungen werden gemittelt.

In jeder Versuchsreihe wird als Referenzsubstanz das Diflucortolon-21-valerianat (= 6$\alpha$,9$\alpha$-Difluor-11$\beta$-hydroxy-16$\alpha$-methyl-21-valeryloxy-1,4-pregnadien-3,20-dion = DFV) verwendet.

Es wird jeweils die Differenz der in den einzelnen Untersuchungsreihen ermittelten mittleren Wirkungsgraden von DFV und Testsubstanz ermittelt. Positive Abweichungen zeigen eine günstigere, negative Abweichungen zeigen eine ungünstigere Testsubstanz im Vergleich zu DFV an.

b) Ermittlung der unerwünschten systemischen Wirksamkeit:

SPF-Ratten im Gewicht von 130 bis 150 g werden zur Erzeugung eines Entzündungsherdes 0,1 ml einer 0,5%-igen Mycobacterium butyricum Suspension (erhältlich von der amerikanischen Firma Difko) in die rechte Hinterpfote injiziert. Vor der Injektion mißt man das Pfotenvolumen der Ratten. 24 Stunden nach der Injektion wird das Pfotenvolumen zur Bestimmung des Ausmaßes des Ödems abermals gemessen. Anschließend appliziert man den Ratten oral unterschiedliche Mengen der Testsubstanz. Nach weiteren 24 Stunden wird das Pfotenvolumen erneut ermittelt.

Die Kontrolltiere werden in gleicher Weise behandelt, mit dem Unterschied, daß ihnen eine testsubstanzfreie Benzylbenzoat-Rhizinusöl-Mischung injiziert wird.

Aus den erhaltenen Pfotenvolumina wird in üblicher Weise die Menge an Testsubstanz bestimmt, welche erforderlich ist, um eine 50%-ige Volumverminderung des experimentell erzeugten Pfotenödems zu erzielen.

In der nachfolgenden Tabelle sind die beobachteten Testergebnisse aufgeführt.

| Nr. | Substanz | Topische Wirksamkeit, Wirkungsgrad DFV — Wirkungsgrad Substanz Konz. der Salbe 0,1 ppm<br>Beobachtungszeit 4 h | <br>Beobachtungszeit 8 h | Systemische Wirksamkeit $ED_{50}$ in mg/kg Tier |
|---|---|---|---|---|
| 1 | DFV | 0,0 definitionsgemäß | 0,0 definitionsgemäß | 0,2 |
| 2 | 17α-Butyryloxy-21-chlor 9α-fluor-16β-methyl-1,4-pregnadien-3,11,20-trion | –0,3 | –0,1 | 8,8 |
| 3 | 17α-Butyryloxy-21-chlor-9α-fluor-1,4-pregnadien-3,11,20-trion | –0,1 | 0,0 | 30 |
| 4 | 17α-Acetoxy-21-chlor-9α-fluor-1,4-pregnadien-3,11,20-trion | –0,1 | –0,1 | 30 |
| 5 | 21-Chlor-9α-fluor-17α-propionyloxy-1,4-pregnadien-3,11,20-trion | 0,0 | –0,2 | 12,0 |
| 6 | 21-Chlor-9α-fluor-17α-valeryloxy-1,4-pregnadien-3,11,20-trion | –0,3 | –0,2 | 30 |

Die neuen Verbindungen eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremes oder Pflaster überführt. In den so formulierten Arzneimitteln ist die Wirkstoffkonzentration von der Applikationsform abhängig. Bei Lotionen und Salben wird vorzugsweise eine Wirkstoffkonzentration von 0,001% bis 1% verwendet.

Darüberhinaus sind die neuen Verbindungen gegebenenfalls in Kombination mit den üblichen Trägermitteln und Hilfsstoffe auch gut zur Herstellung von Inhalationsmitteln geeignet, welche zur Therapie allergischer Erkrankungen der Atemwege, wie zum Beispiel des Bronchialasthmas oder der Rhinitis verwendet werden können.

Die nachfolgenden Beispiele dienen zur Erläuterung der Erfindung.

Beispiel 1

Eine Lösung von 97 ml Methylenchlorid und 7,3 ml Pyridin wird bei 0°C portionsweise mit 3,9 g Chrom(VI)-oxid versetzt und anschließend 15 Minuten bei Raumtemperatur gerührt. Nach erneuter Abkühlung auf 0°C fügt man eine Lösung von 2,7 g 17α-Acetoxy-21-chlor-9α-fluor-11β-hydroxy-1,4-pregnadien-3,20-dion in 54 ml Methylenchlorid und 1 ml Pyridin hinzu und rührt 20 Stunden bei Raumtemperatur weiter. Der abgesaugte Rückstand wird mehrmals mit Methylenchlorid extrahiert und die organische Lösung mit Wasser gewaschen, über Natriumsulfat getrocknet und im Vakuum eingedampft. Die Reinigung des Reaktionsproduktes erfolgt an 300 g Kieselgel mit einem Methylenchlorid-Aceton-Gradienten (0—8% Aceton). Ausbeute 2,19 g 17α-Acetoxy-21-chlor-9α-fluor-1,4-pregnadien-3,11,20-trion. Schmelzpunkt 256°C (unter Zersetzung). $[\alpha]_D^{25} = +101°$ (Chloroform). UV: $\varepsilon_{235}$=16200 (Methanol).

Beispiel 2

Ein Lösung von 750 mg 21-Chlor-9α-fluor-11β-hydroxy-17α-propionyloxy-1,4-pregnadien-3,20-dion in 90 ml Aceton wird bei 0°C unter Rühren mit 7,5 ml Jones-Reagenz versetzt. Es wird 1 Stunde bei 0°C weiter gerührt und anschließend auf Eiswasser gegeben. Man filtriert ab und löst den Rückstand in Methylenchlorid. Die organische Lösung wird mit Wasser neutral gewaschen, über Natriumsulfat getrocknet und im Vakuum eingedampft. Das Rohprodukt von 640 mg wird aus Aceton/Hexan umkristallisiert. Ausbeute 460 mg 21-Chlor-9α-fluor-17α-propionyloxy-1,4-pregnadien-3,11,20-trion. Schmelzpunkt 175°C. $[\alpha]_D^{25} = + 88°$ (Chloroform). UV: $\varepsilon_{235}$=14700 (Methanol).

Beispiel 3

Analog-Beispiel 1 setzt man 2,1 g 17α-Butyryloxy-21-chlor-9α-fluor-11β-hydroxy-1,4-pregnadien-3,20-dion mit Chrom-(VI)-oxid um und reinigt das Rohprodukt an 250 g Kieselgel mit einem Methylenchlorid-Aceton-Gradienten (0—8% Aceton), Ausbeute 1,57 g 17α-Butyryloxy-21-chlor-9α-fluor-1,4-pregnadien-3,11,20-trion. Schmelzpunkt 100°C. $[\alpha]_D^{25} = +92°$ (Chloroform). UV: $\varepsilon_{236}$=15200 (Methanol).

Beispiel 4

Unter den im Beispiel 1 beschriebenen Bedingungen setzt man 1,35 g 21-Chlor-9α-fluor-11β-hydroxy-17α-valeryloxy-1,4-pregnadien-3,20-dion mit Chrom(VI)-oxid um. Das Reaktionsprodukt wird an 150 g Kieselgel mit einem Methylenchlorid-Aceton-Gradienten (0—8% Aceton) gereinigt. Ausbeute 1,08 g 21-Chlor-9α-fluor-17α-valeryloxy-1,4-pregnadien-3,11,20-trion.

Beispiel 5

Man behandelt 1,6 g 21-Chlor-9α-fluor-11β-hydroxy-17α-trimethylacetoxy-1,4-pregnadien-3,20-dion analog Beispiel 2 mit Jones-Reagenz. Die Reinigung des Reaktionsproduktes erfolgt an 200 g Kieselgel mit einem Methylenchlorid-Aceton-Gradienten (o-10% Aceton). Ausbeute 1,47 g 21-Chlor-9α-fluor-17α-trimethylacetoxy-1,4-pregnadien-3,11,20-trion vom Schmelzpunkt 195°C. $[\alpha]_D^{25} = +89°$ (Chloroform).

Beispiel 6

Unter den Bedingungen des Beispiels 1 setzt man 3,3 g 21-Chlor-9α-fluor-11β-hydroxy-17α-isobutyryloxy-1,4-pregnadien-3,20-dion mit Chrom(VI)-oxid um. Das Reaktionsprodukt wird an 300 g Kieselgel mit einem Hexan-Essigester-Gradienten (0—35% Essigester) chromatographiert. Ausbeute 2,65 g 21-Chlor-9α-fluor-17α-isobutyryloxy-1,4-pregnadien-3,11,20-trion. Schmelzpunkt 98°C. $[\alpha]_D^{25} = +90°$ (Chloroform). UV: $\varepsilon_{236}$=15000 (Methanol).

**Patentansprüche für die Vertragsstaaten BE CH DE FR GB IT LU NL SE**

1. Prednison-Derivate der allgemeinen Formel I

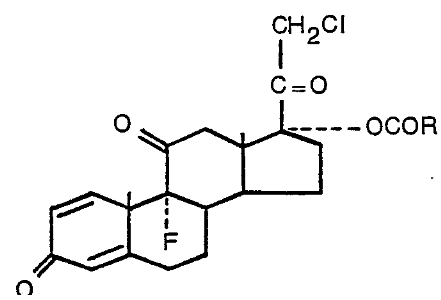

worin
R eine Alkylgruppe mit bis zu 8 Kohlenstoffatomen bedeutet.

2. 17α-Acetoxy-21-chlor-9α-fluor-1,4-pregnadien-3,11,20-trion.
3. 21-Chlor-9α-fluor-17α-propionyloxy-1,4-pregnadien-3,11,20-trion.
4. 17α-Butyryloxy-21-chlor-9α-fluor-1,4-pregnadien-3,11,20-trion.
5. 21-Chlor-9α-fluor-17α-valeryloxy-1,4-pregnadien-3,11,20-trion.
6. 21-Chlor-9α-fluor-17α-trimethylacetoxy-1,4-pregnadien-3,11,20-trion.
7. 21-Chlor-9α-fluor-17α-isobutyryloxy-1,4-pregnadien-3,11,20-trion.
8. Pharmazeutische Präparate gekennzeichnet durch einen Gehalt an 1 bis 3 Prednison-Derivaten gemäß Anspruch 1 bis 7 als Wirkstoff.

9 Verfahren zur Herstellung von Prednison-Derivaten der allgemeinen Formel I gemäß Anspruch 1, dadurch gekennzeichnet, daß man in an sich bekannter Weise
a) die 11β-Hydroxygruppe eines Steroids der allgemeinen Formel II

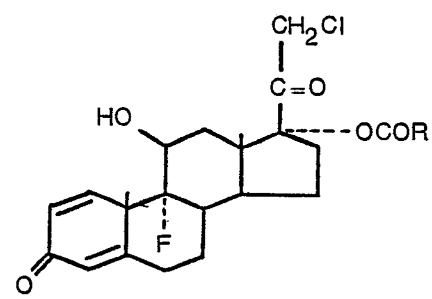

(II)

worin R die im Anspruch 1 genannte Bedeutung besitzt oxydiert, oder

b) die 17-Hydroxygruppe eines Steroids der allgemeinen Formel III

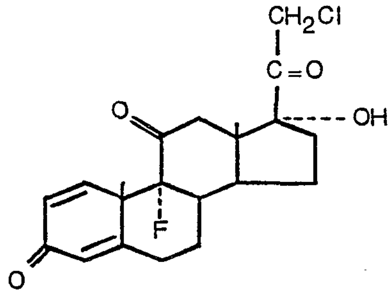

(III)

mit einem reaktionsfähigen Derivat einer Carbonsäure der allgemeinen Formel IV

RCOOH (IV)

worin R die im Anspruch 1 genannte Bedeutung besitzt verestert, oder
c) daß man einen Steroidorthoester der allgemeinen Formel V

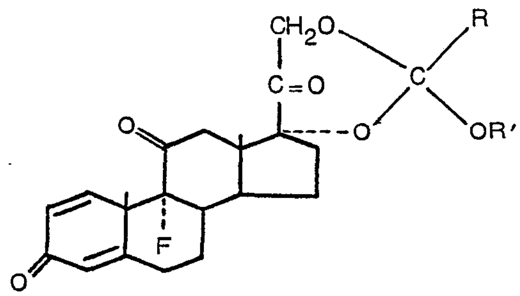

worin R die im Anspruch 1 genannte Bedeutung besitzt und R' eine 1 bis 4 Kohlenstoffatome enthaltende Alkylgruppe darstellt, mit Trimethylsilylchlorid oder Triphenylmethylchlorid öffnet.

**Patentanspruch für den Vertragsstaat AT**

Verfahren zur Herstellung von Prednisolon-Derivaten der allgemeinen Formel I

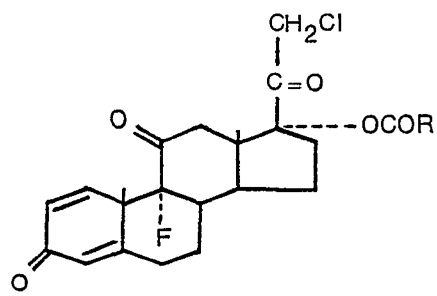

worin
R eine Alkylgruppe mit bis zu 8 Kohlenstoffatomen darstellt, dadurch gekennzeichnet, daß man in an sich bekannter Weise
a) die 11β-Hydroxygruppe eines Steroids der allgemeinen Formel II

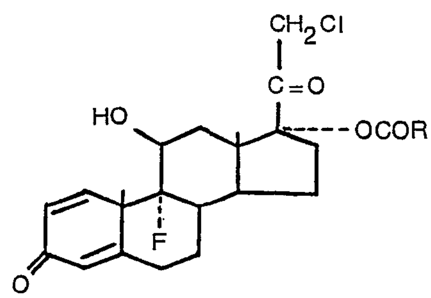

(II)

worin R die obengenannte Bedeutung besitzt oxydiert, oder

b) die 17-Hydroxygruppe eines Steroids der allgemeinen Formel III

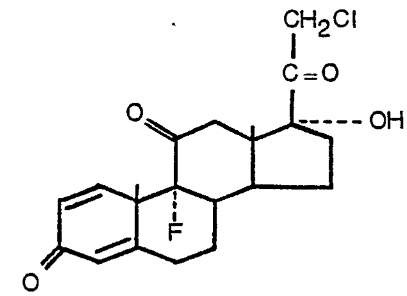

(III)

mit einem reaktionsfähigen Derivat einer Carbonsäure der allgemeinen Formel IV

RCOOH (IV)

worin R die obengenannte Bedeutung besitzt verestert, oder
c) daß man einen Steroidorthoester der allgemeinen Formel V

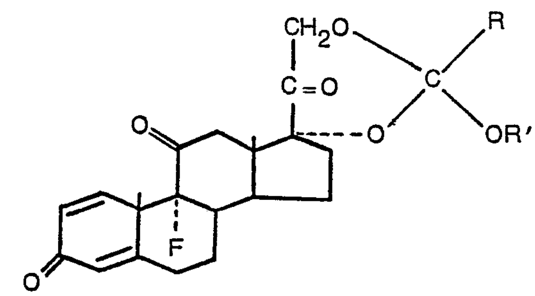

worin R die obengenannte Bedeutung besitzt und R' eine 1 bis 4 Kohlenstoffatome enthaltende Alkylgruppe darstellt, mit Trimethylsilylchlorid oder Triphenylmethylchlorid öffnet.

**Revendications pour les Etats contractants BE CH DE FR GB IT LU NL SE**

1. Dérivés de prédnisone répondant à la formule générale I

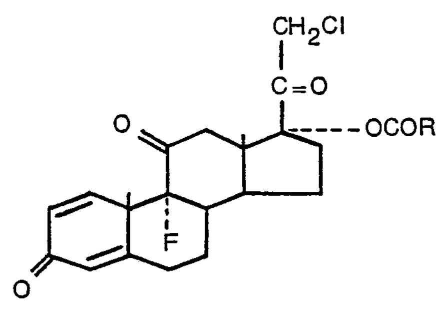

dans laquelle R représente un groupe alkyle ayant jusqu'à 8 atomes de carbone.

2. La 17$\alpha$-acétoxy-21-chloro-9$\alpha$-fluoro-1,4-prégnadiène-3,11,20-trione.
3. La 21-chloro-9$\alpha$-fluoro-17$\alpha$-propionyloxy-1,4-prégnadiène-3,11,20-trione.
4. La 17$\alpha$-butyryloxy-21-chloro-9$\alpha$-fluoro-1,4-prégnadiène-3,11,20-trione.
5. La 21-chloro-9$\alpha$-fluoro-17$\alpha$-valéryloxy-1,4-prégnadiène-3,11,20-trione.
6. La 21-chloro-9$\alpha$-fluoro-17$\alpha$-triméthylacétoxy-1,4-prégnadiène-3,11,20-trione.
7. La 21-chloro-9$\alpha$-fluoro-17$\alpha$-isobutyryloxy-1,4-prégnadiène-3,11,20-trione.
8. Préparations pharmaceutiques caractérisées par une teneur en un à trois dérivés de prédnisone suivant l'une des revendications 1 à 7, comme substance actives.
9. Procédé de préparation de dérivés de prédnisone répondant à la formule générale I de la revendication 1, caractérisé en ce que, d'une manière connue en soi,

a) on oxyde le groupe 11$\beta$-hydroxy d'un stéroïde de la formule générale II

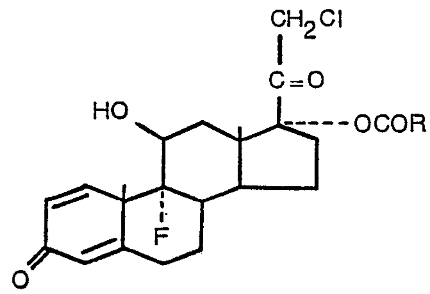

(II)

dans laquelle R a la même signification que celle donnée dans la revendication 1, ou
b) on estérifie le groupe 17-hydroxy d'un stéroïde de la formule générale III

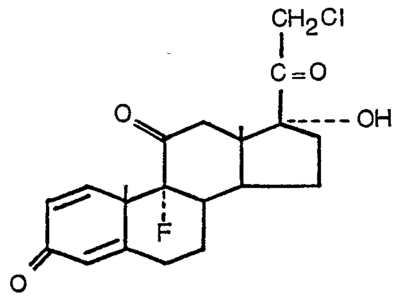

(III)

avec un dérivé réactif d'un acide carboxylique de la formule générale IV

RCOOH (IV)

dans laquelle R a la même signification que celle donnée dans la revendication 1, ou
c) on ouvre un orthoester de stéroïde de la formule générale V

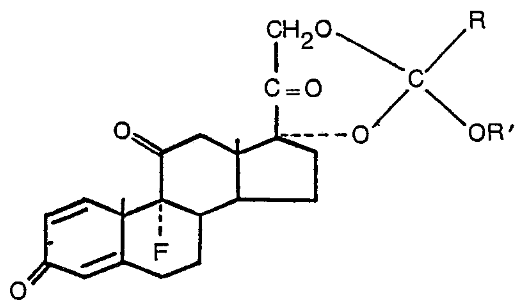

dans laquelle R a la même signification que celle donnée dans la revendication 1 et R' représente un groupe alkyle contenant de 1 à 4 atomes de carbone, avec du chlorure de triméthylsilyle ou du chlorure de triphénylméthyle.

**Revendication pour l'Etat contractant AT**

Procédé de préparation de dérivés de prédnisone répondant à la formule générale I dans laquelle R représente un groupe alkyle ayant jusqu'à 8 atomes de carbone, caractérisé en ce que, d'une manière connue en soi,
a) on oxyde le groupe 11β-hydroxy d'un stéroïde de la formule générale II

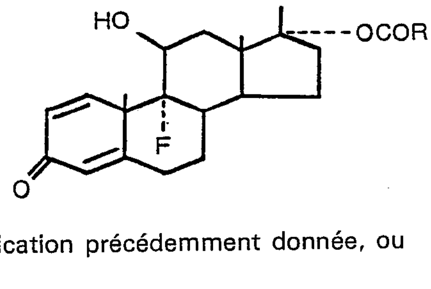

(II)

dans laquelle R a la signification précédemment donnée, ou

b) on estérifie le groupe 17-hydroxy d'un stéroïde de la formule générale III

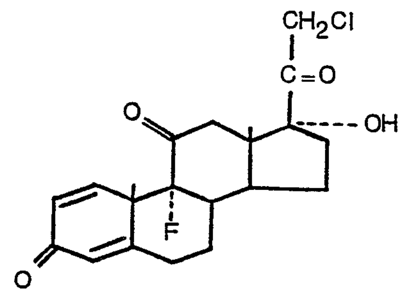

(III)

avec un dérivé réactif d'un acide carboxylique de la formule générale IV

$$RCOOH \qquad (IV)$$

dans laquelle R a la signification précédemment donnée, ou

c) on ouvre un orthoester de stéroïde de la formule générale V

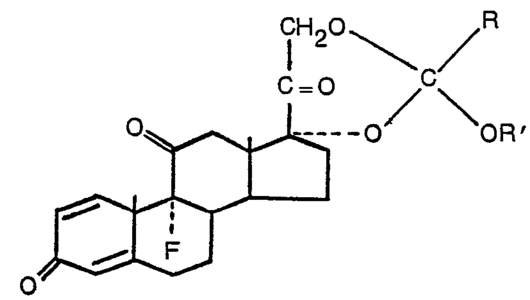

dans laquelle R a la signification précédemment donnée et R' représente un groupe alkyle contenant de 1 à 4 atomes de carbone, avec du chlorure de triméthylsilyle ou du chlorure de triphénylméthyle.

**Claims for the contracting states BE CH DE FR GB IT LU NL SE**

1. Prednisone derivatives of the general formula I

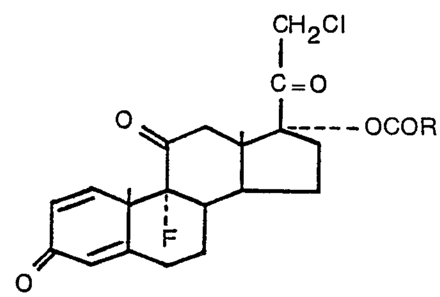

in which

R represents an alkyl group having up to 8 carbon atoms.

2. 17α-acetoxy-21-chloro-9α-fluoro-1,4-pregnadiene-3,11,20-trione.

3. 21-chloro-9α-fluoro-17α-propionyloxy-1,4-pregnadiene-3,11,20-trione.

4. 17α-butyryloxy-21-chloro-9α-fluoro-1,4-pregnadiene-3,11,20-trione.

5. 21-chloro-9α-fluoro-17α-valeryloxy-1,4-pregnadiene-3,11,20-trione.

6. 21-chloro-9α-fluoro-17α-trimethylacetoxy-1,4-pregnadiene-3,11,20-trione.

7. 21-chloro-9α-fluoro-17α-isobutyryloxy-1,4-pregnadiene-3,11,20-trione.

8. Pharmaceutical preparations characterised in that they contain as active substance from 1 to 3 prednisone derivatives according to claims 1 to 7.

9. Process for the manufacture of prednisone derivatives of the general formula I according to claim 1, characterised in that, in a manner known *per se,*

a) the 11β-hydroxy group of a steroid of the general formula II

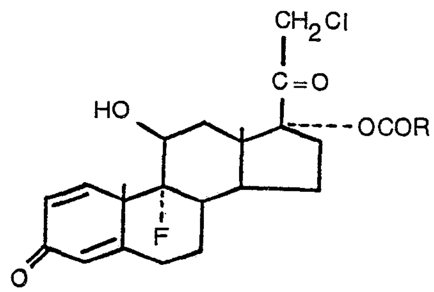

(II)

in which R has the meaning given in claim 1, is oxidised, or
b) the 17-hydroxy group of a steroid of the general formula III

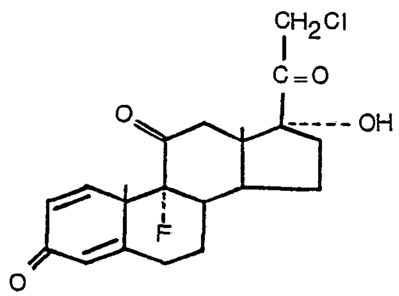

(III)

is esterified with a reactive derivative of a carboxylic acid of the general formula IV

RCOOH (IV)

in which R has the meaning given in claim 1, or
c) a steroid orthoester of the general formula V

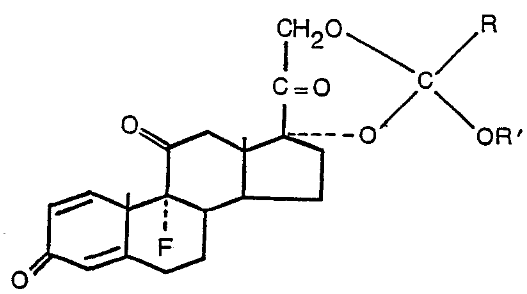

in which R has the meaning given in claim 1 and R′ represents an alkyl group containing from 1 to 4 carbon atoms, is opened with trimethylsilyl chloride or triphenylmethyl chloride.

**Claim for the contracting state AT**

Process for the manufacture of prednisone derivatives of the general formula I

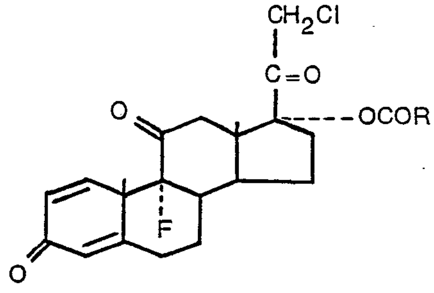

in which
R represents an alkyl group having up to 8 carbon atoms, characterised in that, in a manner known *per se*,

a) the 11β-hydroxy group of a steroid of the general formula II

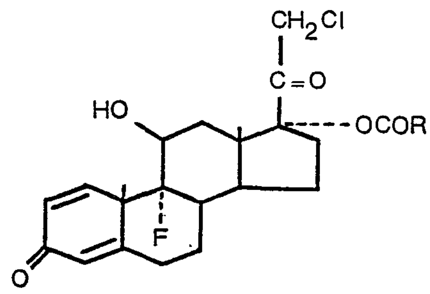

(II)

in which R has the meaning given above, is oxidised, or
b) the 17-hydroxy group of a steroid of the general formula III

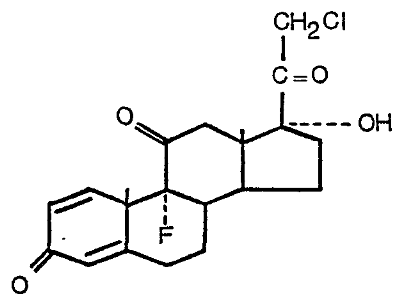

(III)

is esterified with a reactive derivative of a carboxylic acid of the general formula IV

RCOOH (IV)

in which R has the meaning given above, or
c) a steroid orthoester of the general formula V

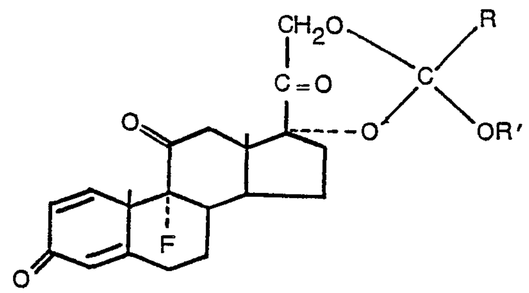

in which R has the meaning given above and R′ represents an alkyl group containing from 1 to 4 carbon atoms, is opened with trimethylsilyl chloride or triphenylmethyl chloride.